# EUROPÄISCHE PATENTSCHRIFT

(11) **EP 4 183 375 B1**
(45) Veröffentlichungstag und Bekanntmachung des Hinweises auf die Patenterteilung: **15.05.2024**
(21) Anmeldenummer: 22206441.2
(22) Anmeldetag: 09.11.2022
(51) Int. Cl.: A61F 2/58, A61F 2/50

(54) **PROTHESENKOSMETIK UND VERFAHREN ZU DESSEN HERSTELLUNG**
PROSTHESIS COSMETIC AND METHOD FOR THE PRODUCTION THEREOF
PRODUIT COSMÉTIQUE PROTHÉTIQUE ET SON PROCÉDÉ DE FABRICATION

(30) Priorität: 17.11.2021 DE 102021130039
(43) Veröffentlichungstag der Anmeldung: 24.05.2023
(73) Patentinhaber: Otto Bock Healthcare Products GmbH, 1110 Wien (AT)
(72) Erfinder: LUNZER, Walter, 1110 Wien (AT); WAGNER, Sonja, 1110 Wien (AT); SCHERB, Alice, 1110 Wien (AT)
(74) Vertreter: Gramm, Lins & Partner Patent- und Rechtsanwälte PartGmbB

(56) Entgegenhaltungen:
- DE-A1-102008 023 006
- DE-A1-102015 006 977
- DE-A1-102016 003 864
- GB-A- 2 067 074
- US-B2- 9 192 486

## Beschreibung

Die Erfindung betrifft eine Prothesenkosmetik mit einem textilen Grundkörper aus einer blickdichten Grundware mit einem ersten Farbton und ein Verfahren zum Herstellen einer Prothesenkosmetik mit einem solchen Grundkörper. US 9 192 486 B2 stellt den nächsten Stand der Technik dar.

Prothesenkosmetiken sind Überzüge aus unterschiedlichen Materialien, die flexibel und in der Regel elastisch sind und über Prothesengliedmaßen oder Prothesenkomponenten getragen werden. Prothesenkosmetiken nehmen eine Schutzfunktion war, indem einerseits die Prothesenkomponenten oder Prothesen Gliedmaßen vor äußeren Einflüssen schützen und andererseits eine Polsterung für natürliche Gliedmaßen darstellen, sollte es während der Nutzung der Prothesen zu einem Kontakt mit natürlichen Gliedmaßen kommen. Neben der Schutzfunktion nehmen Prothesenkosmetiken auch eine ästhetische Funktion war. Häufig wünscht der Nutzer einer Prothese nicht, dass diese als solche unmittelbar erkannt wird. Daher ist es das Ziel von Prothesenkosmetiken, einen an die natürliche Erscheinungsform der Gliedmaße möglichst angepassten und angenäherten optischen Eindruck zu erzeugen.

Prothesenkosmetiken aus einem Elastomermaterial, beispielsweise Silikon, sind schlauchartig ausgebildet oder weisen Aufnahmen für distale Prothesenkomponenten wie Prothesenfüße oder Prothesenhände auf. Die Farbgebung ist dabei außerordentlich aufwendig und wird von Spezialisten in Handarbeit in mehreren Schichten oder Lagen auf einen Elastomer-Grundkörper aufgebracht. Prothesenkosmetiken aus einem Elastomermaterial weisen eine begrenzte Haltbarkeit auf, die Polstereigenschaften sind eingeschränkt, das Material ist schwer und die Herstellung ist mühsam und teuer. Darüber hinaus können Kosmetiken aus Vollsilikon oder Elastomeren den Bewegungsbereich der Prothesen einschränken, beispielsweise bei einer Flexion eines Prothesengelenkes.

Darüber hinaus sind Prothesenkosmetiken aus einem Textilmaterial bekannt, bei denen zur Erzeugung einer möglichst natürlichen Erscheinung das Textilmaterial eingefärbt wird. Die Einfärbung erfolgt entweder mit nur einer Farbe oder unter Aufbringung von Schattierungen. Ebenfalls werden textile Materialien im Rahmen eines Druckverfahrens, beispielsweise Fototransferdruck, bearbeitet, um Hautstrukturen möglichst naturalistisch nachzuahmen. Die dabei erzielten Effekte sind unbefriedigend, da die benötigte Farbtiefe mittels Fotodruckes nicht erreicht werden kann. Die damit hergestellten Produkte wirken künstlich, unnatürlich und werden auf den ersten Blick als orthopädietechnische Produkte erkannt.

Aufgabe der vorliegenden Erfindung ist es, eine Prothesenkosmetik und ein Verfahren zum Herstellen einer Prothesenkosmetik bereitzustellen, mit denen es möglich ist, eine möglichst natürliche optische Erscheinung der Prothesenkosmetik bereitzustellen.

Erfindungsgemäß wird diese Aufgabe durch eine Prothesenkosmetik mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie der Figur offenbart.

Die Prothesenkosmetik mit einem textilen Grundkörper aus einer blickdichten Grundware mit einen ersten Farbton sieht zumindest eine textile Deckware aus einem transparenten, teiltransparenten oder transluzenten Fadenmaterial vor, das auf einer Außenseite der Grundware aufgearbeitet und in einem von dem ersten Farbton abweichenden Farbton oder mit einer davon abweichenden Farbsättigung eingefärbt ist. Bei einem teiltransparenten Fadenmaterial ist der Transmissionsgrad größer als 0,4, insbesondere größer als 0,5. Die Grundware mit dem ersten Farbton weist bevorzugt eine Färbung auf, die der Hautfarbe bzw. dem Farbton der Haut der unversorgten Seite oder des übrigen Körpers ähnelt oder entspricht. Auf der Außenseite dieser Grundware wird eine zweite, textile Deckware aus einem transparenten, teiltransparenten oder transluzenten Fadenmaterial aufgebracht, sodass sich ein verbesserter, tiefer Farbeindruck entsteht, der dem mehrschichtigen Farbeindruck einer natürlichen Haut ähnelt. Mit der erfindungsgemäßen Prothesenkosmetik wird erreicht, dass der natürliche Hautschimmer ohne aufwändige Drucke, künstliche Schattierungen oder andere Maßnahmen mit einem textilen Werkstoff erreicht wird, sodass eine leichte, preiswerte und einfach herzustellende Prothesenkosmetik bereitgestellt wird. Die blickdichte, undurchsichtige Grundware verhindert, dass eine direkte optische Wahrnehmung der abgedeckten Prothesenkomponente erfolgen kann. Durch die dem Farbton der Haut angenäherte Farbgebung der Grundware wird die optische Ähnlichkeit zu einer natürlichen Gliedmaße grundsätzlich hergestellt, die darüber angeordnete zweite Materialschicht aus einem Textil vermeidet einen stumpfen Eindruck und ermöglicht aufgrund des textilen Charakters eine Faltenbildung bei der Bewegung der Prothesengliedmaße.

Eine Weiterbildung sieht vor, dass die Grundware als Gestrick, Gewirk oder Gewebe ausgebildet ist und die Deckware über ein mehrbettiges Strickverfahren, Wirkverfahren oder Webverfahren auf der Grundware aufgearbeitet ist. Dadurch kann einfach und kostengünstig das textile Grundmaterial als Rohware hergestellt werden, aus dem dann die Prothesenkosmetik gefertigt wird. Die Prothesenkosmetik kann aus Zuschnitten gefertigt oder einstückig ausgebildet sein.

Eine Weiterbildung sieht vor, dass die Grundware ein erstes Fadenmaterial aufweist, insbesondere daraus besteht, beispielsweise Polyester oder Viskose. Das erste Fadenmaterial ist insbesondere opak und von dem transparenten, teiltransparenten oder transluzenten Fadenmaterial der Deckware verschieden. Bevorzugt besteht das zweite Fadenmaterial aus Nylon, insbesondere aus einem hochglatten Nylon oder Polyamid und ist als Monofile oder Monofilament ausgebildet. Durch die Verwendung unterschiedlicher Fadenmaterialien ist die Ausgestaltung der Prothesenkosmetik entsprechend ihrer Funktion verbessert einstellbar. Die der Prothese zugewandte Grundware aus dem ersten Fadenmaterial oder mit dem ersten Fadenmaterial weist insbesondere einen dunkleren Farbton auf die Deckware aus einem zweiten Fadenmaterial. Die Ausgestaltung des ersten Fadenmaterials beispielsweise aus Polyester oder Viskose ermöglicht eine leichte Einfärbung und eine hohe Farbbeständigkeit der Grundware, während die Verwendung des zweiten Fadenmaterials aus Nylon eine optisch und haptisch ansprechende Außenoberfläche der Prothesenkosmetik bereitstellt. Die zwei unterschiedlichen Farben der Grundware und der Deckware in Kombination mit den transparenten oder transluzenten Eigenschaften des Materials der Deckware verleihen dem Textil der Prothesenkosmetik eine Tiefenwirkung, die dem optischen Eindruck der menschlichen Haut angenähert ist.

Eine Weiterbildung sieht vor, dass die Grundware aus mehreren Fadenmaterialien gebildet ist, die unterschiedliche Fadenstärken und/oder Materialeigenschaften aufweisen. Die Verwendung mehrerer Fadenmaterialien mit unterschiedlichen Fadenstärken und/oder Materialeigenschaften vergrößern die Gestaltungsmöglichkeiten hinsichtlich des optischen Eindruckes sowie der funktionalen Eigenschaften. Beispielsweise können unterschiedlich dicke Garne verwendet werden, um unterschiedliche Polstereigenschaften, elastische Eigenschaften, mechanische Widerstände und erhöhte Blickdichtigkeiten zu erzeugen. Die Grundware kann mit einer höheren Dichte als die Deckware erzeugt werden, sodass die Deckware eine erhöhte Nachgiebigkeit und Polsterwirkung insbesondere aufgrund der Verwendung von Monofile-Garnen erzeugt, während die Grundware eine mechanische Festigkeit und die optische Basis für den gewählten Farbton gewährleistet.

Eine Weiterbildung sieht vor, dass in der Grundware zumindest ein elastisches Fadenmaterial eingearbeitet ist oder die Grundware eine Elastizität von mindestens 100% aufweist, wobei zu der Bestimmung der Elastizität bevorzugt das Verfahren der mehrmaligen Zugbeanspruchung nach der DIN 53835 T1 oder DIN 53835 T3 oder beider dieser Verfahren angewendet wird. Unabhängig von dem Messverfahren wird in jeder Variante eine Elastizität von 100% nachgewiesen. Eine elastische Grundware erleichtert das Anlegen der Prothesenkosmetik über der Prothesenkomponente und die Faltenbildung sowie Rückstellung bei einer Verlagerung von Prothesenkomponenten relativ zueinander oder zu der Gliedmaße, an der die Prothese und die Prothesenkosmetik befestigt sind. Dadurch werden die elastischen Eigenschaften der Haut nachgebildet und eine verbesserte natürliche Erscheinungsform erzeugt. Eine Elastizität von mehr als 100 % ist in der Regel ausreichend, um ansprechende optische und mechanische Eigenschaften zu erzeugen, größere Elastizitäten sind ebenfalls möglich und gewünscht.

Eine Weiterbildung sieht vor, dass mehrere Decklagen übereinander an der Außenseite der Grundware angeordnet sind, um eine größere optische Tiefe zu erreichen. Die jeweilige Lage der Deckware an der Außenseite der Grundware kann individuell gefärbt und eine von den übrigen Lagen abweichende Färbung und/oder Materialeigenschaft aufweisen. So können Materialien mit unterschiedlichen Transmissionsgraden je Lage der Deckware verwendet werden, wobei vorteilhafterweise der Transmissionsgrad für jede Lage in Richtung auf die Grundware verringert wird. Eine Lage der Deckware kann auch unterschiedliche Farbtöne oder Farbwerte aufweisen, sodass in bestimmten Bereichen der Prothesenkosmetik Schattierungen erzeugt werden, die das natürliche Erscheinungsbild verbessern

Eine Weiterbildung sieht vor, dass die Fadenmaterialien silikonisiert und/oder hydrophobiert sind, insbesondere werden die Garne oder Fadenmaterialien erst nach dem Färben silikonisiert und/oder hydrophobiert. Dies verändert die mechanischen Eigenschaften und den optischen Eindruck und verbessert die Farbstabilität und die Nutzungseigenschaften der Prothesenkosmetik im Endzustand.

Eine Weiterbildung sieht vor, dass die Garnstärke der Grundware abweichend von der Garnstärke der Deckware ist, insbesondere ist die Garnstärke der Grundware größer als die Garnstärke der Deckware, um eine möglichst geschlossene, glatte und feine Außenoberfläche bereitzustellen, während die Grundware eine mechanisch haltbare und dichte Basis bildet.

In einer Ausgestaltung ist das Fadenmaterial der Grundware aus mehreren Filamenten zusammengesetzt, alternativ oder ergänzend besteht das Fadenmaterial der Deckware aus einem Monofilament.

Das Verfahren zum Herstellen einer wie oben beschriebenen Prothesenkosmetik sieht vor, dass zunächst eine Rohware mit einer Grundware und einer Deckware aus unterschiedlichen Fadenmaterialien hergestellt und die Rohware anschließend gefärbt und dann zu einer Prothesenkosmetik verarbeitet wird. Die Herstellung der Rohware vor dem Färben ermöglicht eine einfache Anpassung vorbereiteter und vorgefertigter Materialien an den jeweils gewünschten Farbton durch nachträgliches Färben. Aufgrund der unterschiedlichen Materialeigenschaften der verschiedenen Fadenmaterialien für die Grundware und die Deckware ergeben sich nach dem gemeinsamen Färben Farbeffekte, die dem Erscheinungsbild der natürlichen Haut nahekommen oder entsprechen. Transparente, teiltransparente oder transluzente Fadenmaterialien der Deckware nehmen die Farbe bei der Einfärbung schlechter an als die Materialien der Grundware, insbesondere wenn das Fadenmaterial der Deckware ein Monofilament aus einem Polyamid ist. Insbesondere bei einer hochglatten Variante der Garne der Deckware ergibt sich eine beabsichtigte, schlechtere Bindung der Farbe an der Oberfläche oder in dem Aufbau des Materials, sodass der schimmernde Farbeffekt durch übereinander gelagerte Farbschichten erreicht wird.

Das transparente, teiltransparente oder transluzente Fadenmaterial kann vor der Verarbeitung zu der Deckware voreingefärbt, insbesondere durch Spinndüseneinfärbung gefärbt werden, sodass sich weitere Farbeffekte und Farbüberlagerungen einstellen. Die Voreinfärbung kann ungleichmäßig sein, um unterschiedliche Strukturen der Hautoberfläche optisch nachzubilden. Die Voreinfärbung erfolgt insbesondere mit einer anderen Farbe oder in einem anderen Farbton als bei der Endfärbung.

Bevorzugt wird die Rohware vor der Weiterverarbeitung silikonisiert und/oder hydrophobiert, um die Haltbarkeit und Widerstandsfähigkeit gegenüber äußeren Einflüssen zu erhöhen.

Nachfolgend wird ein Ausführungsbeispiel der Erfindung anhand der Figuren näher erläutert. Es zeigen:
Figur 1 - eine schematische Schnittansicht durch eine Rohware;
Figur 2 - ein schematisches Anwendungsbeispiel einer Prothesenkosmetik eine Prothesenhand;
Figur 3 - zwei Ansichten könne Prothesenkosmetik mit aufgesetzten Fingerkuppen;
Figur 4 - eine Darstellung der Fingerüberzüge allein;
Figur 5 eine Darstellung einer Prothesenkosmetik als Prothesenhand Überzug; sowie
Figur 6 - eine schematische Darstellung einer Prothesenkosmetik als Prothesenhandüberzug.

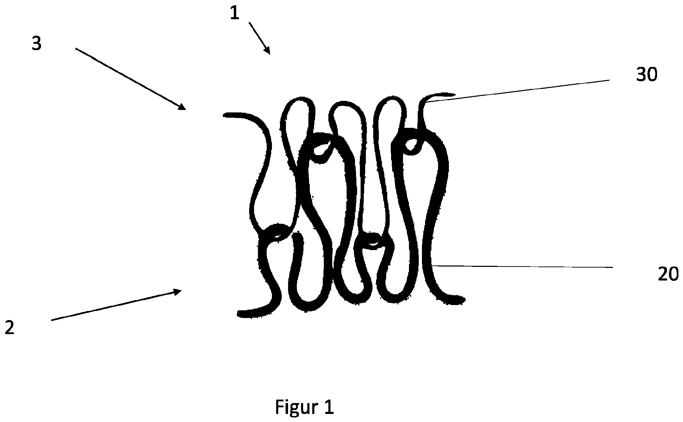

In der Figur 1 ist eine schematische Schnittansicht durch eine Rohware 1 einer Prothesenkosmetik gezeigt. Die Rohware 1 besteht in dem dargestellten Ausführungsbeispiel aus einer Grundware 2 und einer Deckware 3. Die Grundware 2 ist als Gestrick, Gewirk oder Gewebe ausgebildet und weist ein Garn aus einem texturierten Polyester mit einem plattierten Elastomergarn als erstes Fadenmaterial 20 auf. Das Garn oder Fadenmaterial 20 der Grundware 2 erzeugt eine blickdichte Schicht oder Basis und ist insbesondere als ein Multifilamentengarn ausgebildet, mit einem Massenanteil von mindestens 5% Elastomergarn und maximal 95% Polymergarn.

In der Deckware 3 wird ein Garn als Fadenmaterial 30 verwendet, das aus einem transparenten oder teiltransparenten Polyamid besteht. Anders als das Fadenmaterial 20 der Grundware 2 wird das Fadenmaterial 30 der Deckware 3 durch ein gemeinsames Färbebad nur leicht angefärbt und erhält dadurch eine Tönung, die sich je nach vor Färbung von dem Farbeindruck der Grundware 2 unterscheidet.

Die Rohware 1 wird zunächst vollständig hergestellt, beispielsweise durch ein gemeinsames Stricken, Wirken oder Weben. Alternativ zur einer einlagigen Ausgestaltung der Deckschicht 3 kann diese auch mehrlagig ausgestaltet sein. Bei der Herstellung der Rohware 1 wird diese gemeinsam gefärbt und anschließend weiterverarbeitet. Die Rohware 1 kann elastische Eigenschaften aufweisen und bei einem Einsatz als hülsenartige Prothesenkosmetik in einem Rahmen eines Rundstrickverfahrens einstückig hergestellt sein.

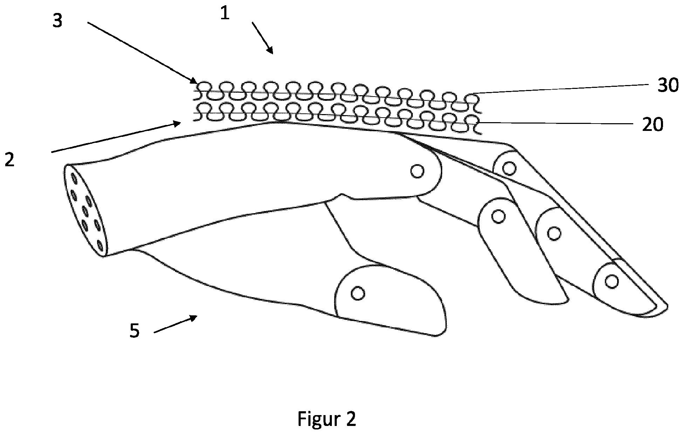

in der Figur 2 ist einer schematischen Darstellung eine Prothesenhand 5 mit einem Grundkörper und mehreren daran gelenkig befestigten Prothesenfingern gezeigt. Prothesenfinger weisen mehrere Komponenten auf, die gelenkig aneinander befestigt sind. Zur Bewegung der Prothesenfinger relativ zu dem Grundkörper können Antriebe, beispielsweise Elektromotoren, in dem Grundkörper vorhanden sein, so dass die Prothesenhand 5 als aktive Prothese mit einer entsprechenden Steuerung, beispielsweise auf der Grundlage myoelektrische Signale, eingesetzt werden kann. An der Außenseite der Prothesenhand 5 ist ein Teil einer Prothesenkosmetik oder eines Prothesenüberzuges aus einer Rohware 1 gezeigt, die auf der der Prothesenhand 5 zugewandten Unterseite eine blickdichte Grundware 2 aus einem Gestrick, Gewirk oder Gewebe aufweist, auf dessen der Prothesenhand 5 abgewandten Außenseite eine Deckware 3 aus einem transparenten, transluzenten oder teiltransparenten Gestrick, Gewirk oder Gewebe aufgebracht ist. Der Aufbau des Materials der Rohware 1 entspricht dem in der Figur 1 beschriebenen Aufbau. Die Rohware 1 ist an der Außenseite der Prothesenhand 5 angeordnet, beispielsweise als alleiniger Prothesenüberzug oder Prothesenkosmetik oder aber als Teil einer Kombination mehrerer Komponenten.

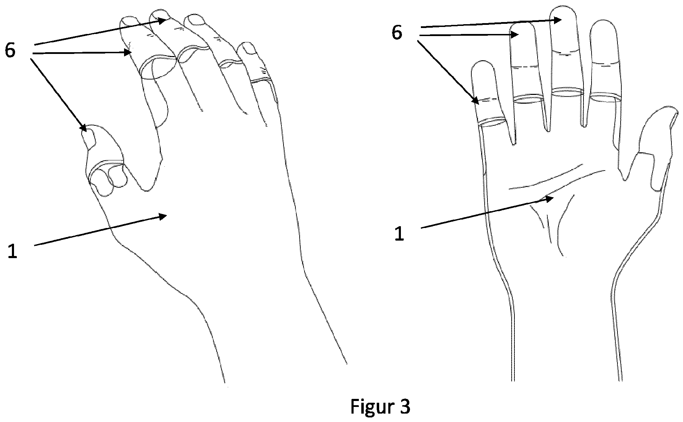

In der Figur 3 ist eine weitere Variante der Erfindung gezeigt, bei der ein Prothesenüberzug oder eine Prothesenkosmetik aus einer Rohware 1 in Gestalt eines Teilhandschuhes ohne Fingerkuppen oder nur mit teilweise ausgebildeten Fingerkuppen dargestellt ist. An den distalen Enden der Prothesenkosmetik sind aufgeklebte Spritzgussteile oder Elastomerkomponenten fest angeordnet, die für den jeweiligen Einsatzzweck die notwendigen Materialeigenschaften aufweisen, beispielsweise hinsichtlich der Verschleißfestigkeit und/oder der Haftfähigkeit. Darüber hinaus können die Elastomerkomponenten 6 zur Verbesserung der natürlichen Erscheinung des Prothesenüberzuges Fingernagelkomponenten aufweisen.

Aufgrund der bereits vorher eingefärbten Garne der Grundware 2 und der Deckware 3 ist eine weitergehende oder intensive Farbgebung der Elastomerkomponenten 6 nicht notwendig, allerdings kann durch eine Einfärbung der Elastomerkomponenten 6 eine Gesamtveränderung des Farbeindruckes hervorgerufen werden. Neben der Anordnung der Elastomerkomponenten 6 an den Fingerkuppen kann dies auch an dem Handrücken und/oder an der Handflächeninnenseite angeordnet und aufgebracht werden. Die Elastomerkomponenten werden entweder aufgesteckt oder aufgeklebt, angegossen oder sind als vollständiger Handüberzug ausgebildet und werden über die Textilware übergezogen.

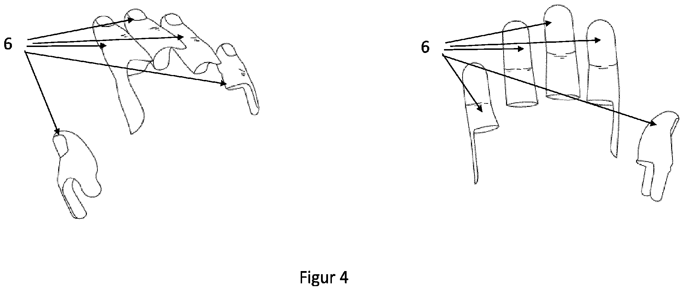

In der Figur 4 sind die insgesamt fünf Elastomerkomponenten 6 in Gestalt von Fingerkuppen in Einzeldarstellung dargestellt. Neben den reinen Fingerkuppen, die das distale und mediale Fingerglied abdecken bzw. das Daumenendglied, sind im Bereich des Zeigefingers und des kleinen Fingers mediale und laterale Verlängerungen ausgebildet, um besonders verschleißanfällige Bereiche des textilen Grundmaterials abzudecken und zu schützen.

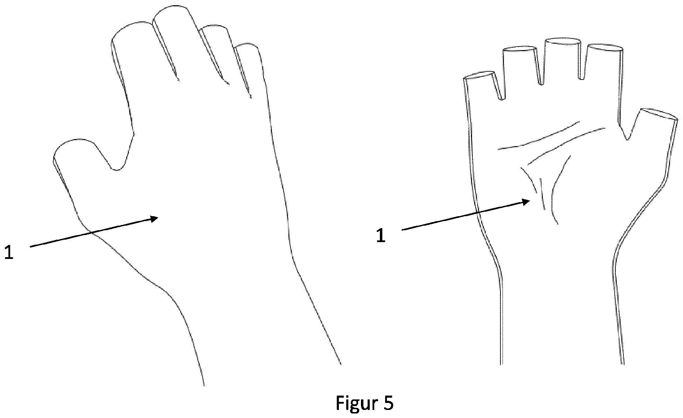

In der Figur 5 ist der Überzug mit der Rohware 1 aus der Grundware 2 und der Deckware 3 in Gestalt eines Teilhandschuhes gezeigt, bei dem die Fingerkuppen vollständig frei sind.

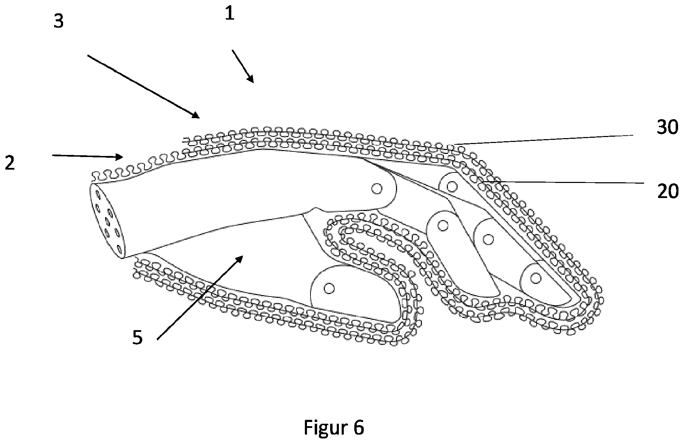

In der Figur 6 ist der Überzug als Prothesenhandkosmetik mit der Rohware 1 aus der Grundware 2 mit dem ersten Fadenmaterial 20 und der Deckware 3 mit dem zweiten Fadenmaterial 30 aus einem transparenten oder teiltransparenten bzw. transluzenten Material, insbesondere Polyamid gezeigt.

## Patentansprüche

1. Prothesenkosmetik mit einem textilen Grundkörper aus einer blickdichten Grundware (2) mit einen ersten Farbton und zumindest einer textilen Deckware (3) aus einem transparenten, teiltransparenten oder transluzenten Fadenmaterial (30), das auf einer Außenseite der Grundware (2) aufgearbeitet ist und in einem von dem ersten Farbton abweichenden Farbton oder mit einer davon abweichenden Farbsättigung eingefärbt ist.

2. Prothesenkosmetik nach Anspruch 1, **dadurch gekennzeichnet, dass** die Grundware (2) als Gestrick, Gewirk oder Gewebe ausgebildet ist und die Deckware (3) über ein mehrbettiges Strick-, Wirk- oder Webverfahren auf der Grundware (2) aufgearbeitet ist.

3. Prothesenkosmetik nach Anspruch 1 oder 2, **dadurch gekennzeichnet, dass** die Grundware (2) ein erstes Fadenmaterial (20) aufweist, das von dem transparente, teiltransparenten oder transluzenten Fadenmaterial (30) der Deckware (3) verschieden ist.

4. Prothesenkosmetik nach einem der voranstehenden Ansprüche, **dadurch gekennzeichnet, dass** die Grundware (2) aus mehreren Fadenmaterialien (20) gebildet ist, die unterschiedliche Fadenstärken und/oder Materialeigenschaften aufweisen.

5. Prothesenkosmetik nach einem der voranstehenden Ansprüche, **dadurch gekennzeichnet, dass** in der Grundware (2) zumindest ein elastisches Fadenmaterial eingearbeitet ist oder die Grundware (2) eine Elastizität von mindestens 100% aufweist.

6. Prothesenkosmetik nach einem der voranstehenden Ansprüche, **dadurch gekennzeichnet, dass** mehrere Decklagen übereinander an der Außenseite der Grundware (2) angeordnet sind.

7. Prothesenkosmetik nach einem der voranstehenden Ansprüche, **dadurch gekennzeichnet, dass** die Fadenmaterialien (20, 30) silikonisiert und/oder hydrophobiert sind.

8. Prothesenkosmetik nach einem der voranstehenden Ansprüche, **dadurch gekennzeichnet, dass** die Garnstärke der Grundware (2) abweichend von der Garnstärke der Deckware (3) ist.

9. Prothesenkosmetik nach einem der voranstehenden Ansprüche, **dadurch gekennzeichnet, dass** das Fadenmaterial (20) der Grundware (2) aus mehreren Filamenten zusammengesetzt ist und/oder das Fadenmaterial (30) der Deckware (3) aus einem Monofilament besteht.

10. Verfahren zum Herstellen einer Prothesenkosmetik nach einem der voranstehenden Ansprüche, **dadurch gekennzeichnet, dass** zunächst eine Rohware (1) mit einer Grundware (2) und einer Deckware (3) aus unterschiedlichen Fadenmaterialien (20, 30) hergestellt und die Rohware (1) anschließend gefärbt und zu einer Prothesenkosmetik verarbeitet wird.

11. Verfahren nach Anspruch 10, **dadurch gekennzeichnet, dass dadurch gekennzeichnet, dass** das transparente, teiltransparente oder transluzente Fadenmaterial (20) vor der Verarbeitung zu der Deckware (3) voreingefärbt wird.

12. Verfahren nach Anspruch 11, **dadurch gekennzeichnet, dass** die Voreinfärbung mit einer anderen Farbe oder in einem anderen Farbton als bei der Endfärbung erfolgt.

13. Verfahren nach einem der Ansprüche 10 bis 12, **dadurch gekennzeichnet, dass** die Grundware (2) vor der Weiterverarbeitung silikonisiert und/oder hydrophobiert wird.

## Claims

1. Prosthesis cosmetic with a textile base body made of an opaque base fabric (2) with a first colour shade and at least one textile cover fabric (3) made of a transparent, partially transparent or translucent thread material (30), which is finished on an outer side of the base fabric (2) and is coloured in a colour shade differing from the first colour shade or with a colour saturation differing therefrom.

2. Prosthetic cosmetic according to claim 1, **characterised in that** the base fabric (2) is formed as a knitted, warp-knitted or woven fabric and the cover fabric (3) is worked onto the base fabric (2) by a multi-bed knitting, warp-knitting or weaving process.

3. Prosthetic cosmetic according to claim 1 or 2, **characterised in that** the base fabric (2) has a first thread material (20) which is different from the transparent, partially transparent or translucent thread material (30) of the cover fabric (3).

4. Prosthetic cosmetic according to one of the preceding claims, **characterised in that** the base fabric (2) is formed from a plurality of thread materials (20) which have different thread thicknesses and/or material properties.

5. Prosthetic cosmetic according to one of the preceding claims, **characterised in that** at least one elastic thread material is incorporated in the base fabric (2) or the base fabric (2) has an elasticity of at least 100%.

6. Prosthetic cosmetic according to one of the preceding claims, **characterised in that** several cover layers are arranged one above the other on the outside of the base article (2).

7. Prosthesis cosmetic according to one of the preceding claims, **characterised in that** the thread materials (20, 30) are siliconised and/or hydrophobised.

8. Prosthesis cosmetic according to one of the preceding claims, **characterised in that** the yarn weight of the base fabric (2) is different from the yarn weight of the cover fabric (3).

9. Prosthetic cosmetic according to one of the preceding claims, **characterised in that** the thread material (20) of the base fabric (2) is composed of several filaments and/or the thread material (30) of the cover fabric (3) consists of a monofilament.

10. Method for producing a prosthesis cosmetic according to one of the preceding claims, **characterised in that** a raw material (1) is first produced with a base material (2) and a cover material (3) from different thread materials (20, 30) and the raw material (1) is then pre-coloured and processed into a prosthesis cosmetic.

11. Method according to claim 10, **characterised in that** the transparent, partially transparent or translucent thread material (20) is pre-coloured before processing into the cover fabric (3).

12. Process according to claim 11, **characterised in that** the pre-colouring is carried out with a different colour or in a different shade than the final colouring.

13. Method according to one of claims 10 to 12, **characterised in that** the base material (2) is siliconised and/or hydrophobised before further processing.

## Revendications

1. Elément esthétique pour prothèse, comprenant un corps de base textile constitué d'une base opaque (2), ayant une première nuance de couleur, et d'au moins une couverture textile (3) constituée d'un matériau de fil transparent, partiellement transparent ou translucide (30) qui est travaillé sur une face extérieure de la base (2) et qui est teinté dans une nuance de couleur différente de la première nuance de couleur ou avec une saturation de couleur différente de celle-ci.

2. Elément esthétique pour prothèse selon la revendication 1,
**caractérisé en ce que** la base (2) est réalisée sous forme de tricot, de maillage ou de tissu, et la couverture (3) est travaillée sur la base (2) par un procédé de tricotage, de maillage ou de tissage à plusieurs fontures.

3. Elément esthétique pour prothèse selon la revendication 1 ou 2, **caractérisé en ce que** la base (2) comprend un premier matériau de fil (20) qui est différent du matériau de fil transparent, partiellement transparent ou translucide (30) de la couverture (3).

4. Elément esthétique pour prothèse selon l'une des revendications précédentes,
**caractérisé en ce que** la base (2) est formée de plusieurs matériaux de fil (20) qui présentent des épaisseurs de fil et/ou des propriétés de matériau différentes.

5. Elément esthétique pour prothèse selon l'une des revendications précédentes,
**caractérisé en ce qu'**au moins un matériau de fil élastique est incorporé dans la base (2), ou la base (2) présente une élasticité d'au moins 100 %.

6. Elément esthétique pour prothèse selon l'une des revendications précédentes,
**caractérisé en ce que** plusieurs couches de couverture sont disposées les unes au-dessus des autres sur la face extérieure de la base (2).

7. Elément esthétique pour prothèse selon l'une des revendications précédentes,
**caractérisé en ce que** les matériaux de fil (20, 30) sont siliconés et/ou rendus hydrophobes.

8. Elément esthétique pour prothèse selon l'une des revendications précédentes,
**caractérisé en ce que** l'épaisseur de fil da la base (2) est différente de l'épaisseur de fil de la couverture (3).

9. Elément esthétique pour prothèse selon l'une des revendications précédentes,
**caractérisé en ce que** le matériau de fil (20) de la base (2) est composé de plusieurs filaments, et/ou le matériau de fil (30) de la couverture (3) est constitué d'un monofilament.

10. Procédé de fabrication d'un élément esthétique pour prothèse selon l'une des revendications précédentes,
**caractérisé en ce que** l'on fabrique d'abord un produit brut (1) comprenant une base (2) et une couverture (3) constituées de différents matériaux de fil (20, 30), puis on teint le produit brut (1) et on le traite pour donner un élément esthétique pour prothèse.

11. Procédé selon la revendication 10,
**caractérisé en ce que** le matériau de fil transparent, partiellement transparent ou translucide (20) est pré-teinté avant d'être traité pour donner la couverture (3).

12. Procédé selon la revendication 11,
**caractérisé en ce que** la pré-teinture est effectuée avec une couleur ou une nuance de couleur différente de celle de la teinture finale.

13. Procédé selon l'une des revendications 10 à 12,
**caractérisé en ce que** la base (2) est siliconée et/ou rendue hydrophobe avant le traitement ultérieur.
